# EUROPEAN PATENT APPLICATION

(11) **EP 4 666 947 A2**
(43) Date of publication of application: **24.12.2025**
(21) Application number: 25198877.0
(22) Date of filing: 26.05.2022
(51) Int. Cl.: A61B 5/367

(54) **CATHETERS FOR MAPPING AND ELECTROPORATION ABLATION**

(30) Priority: 28.05.2021 US 202163194689 P
(62) Divisional of application: 22732796.2
(71) Applicant: Boston Scientific Scimed, Inc., Maple Grove, MN 55311 (US)
(72) Inventor: MAIERHOFER, Edward J., Brooklyn Park, MN 55443 (US); ACHTERHOFF, Trey H., St Paul, MN 55104 (US); KOOP, Brendan E., Ham Lake, MN 55304 (US)
(74) Representative: Pfenning, Meinig & Partner mbB

(57) **Abstract**

Various aspects of the present disclosure are directed toward apparatuses, systems, and methods for electroporation ablation. The electroporation catheter may include an electrode assembly comprising one or more ablation electrodes configured to generate electric fields proximate to target tissue in response to a plurality of electrical pulse sequences delivered in a plurality of therapy sections, and one or more mapping electrodes configured to measure cardiac electrical signals. In some embodiments, the measured electrical signals are used to create an electro-anatomical map.

## Description

### CROSS REFERENCE TO RELATED APPLICATION

This application claims priority to Provisional Application No. 63/194,689, filed May 28, 2021, which is herein incorporated by reference in its entirety.

### TECHNICAL FIELD

The present disclosure relates to medical apparatus, systems, and methods for mapping and applying irreversible electroporation pulse trains. More specifically, the present disclosure relates to an electroporation catheter including one or more electrodes for mapping purpose.

### BACKGROUND

Ablation procedures are used to treat many different conditions in patients. Ablation may be used to treat cardiac arrhythmias, benign tumors, cancerous tumors, and to control bleeding during surgery. Usually, ablation is accomplished through thermal ablation techniques including radio-frequency (RF) ablation and cryoablation. In RF ablation, a probe is inserted into the patient and radio frequency waves are transmitted through the probe to the surrounding tissue. The radio frequency waves generate heat, which destroys surrounding tissue and cauterizes blood vessels. In cryoablation, a hollow needle or cryoprobe is inserted into the patient and cold, thermally conductive fluid is circulated through the probe to freeze and kill the surrounding tissue. RF ablation and cryoablation techniques indiscriminately kill tissue through cell necrosis, which may damage or kill otherwise healthy tissue, such as tissue in the esophagus, phrenic nerve cells, and tissue in the coronary arteries.

Another ablation technique uses electroporation. In electroporation, or electro-permeabilization, an electric field is applied to cells to increase the permeability of the cell membrane. The electroporation may be reversible or irreversible, depending on the strength of the electric field. If the electroporation is reversible, the increased permeability of the cell membrane may be used to introduce chemicals, drugs, and/or deoxyribonucleic acid (DNA) into the cell, prior to the cell healing and recovering. If the electroporation is irreversible, the affected cells are killed through apoptosis.

Irreversible electroporation (IRE) may be used as a nonthermal ablation technique. In IRE, trains of short, high voltage pulses are used to generate electric fields that are strong enough to kill cells through apoptosis. In ablation of cardiac tissue, IRE may be a safe and effective alternative to the indiscriminate killing of thermal ablation techniques, such as RF ablation and cryoablation. IRE may be used to kill target tissue, such as myocardium tissue, by using an electric field strength and duration that kills the target tissue but does not permanently damage other cells or tissue, such as non-targeted myocardium tissue, red blood cells, vascular smooth muscle tissue, endothelium tissue, and nerve cells.

During the course of IRE therapy sections, it is desirable for a catheter that can both create a map and apply IRE pulse trains, for example, for cost sensitive cases. In other words, there is need for a catheter that can provide multiple mapping electrodes as well as a bi-polar and/or mono-polar conduction path for IRE pulse trains.

### SUMMARY

In Example 1, an electroporation catheter comprises an electrode assembly. The electrode assembly comprises one or more ablation electrodes configured to generate electric fields proximate to target tissue in response to a plurality of electrical pulse sequences delivered in a plurality of therapy sections, and one or more mapping electrodes configured to measure electrical signals of a heart, the measured electrical signals being used to create an electro-anatomical map.

In Example 2, the electroporation catheter of Example 1 further comprises a catheter shaft having a proximal end and a distal end, wherein the electrode assembly extends from the distal end of the catheter shaft.

In Example 3, the electroporation catheter of any of Examples 1-2, wherein the electrode assembly further comprises a plurality of splines.

In Example 4, the electroporation catheter of Example 3, wherein a mapping electrode of the one or more mapping electrodes is disposed on a first surface of a spline of the plurality of splines, and an ablation electrode of the one or more ablation electrodes is disposed on a second surface of the spline of the plurality of splines.

In Example 5, the electroporation catheter of any of Examples 2-4, wherein the ablation electrode is disposed further from the distal end of the catheter shaft than the mapping electrode.

In Example 6, the electroporation catheter of any of Example 3-5, wherein each spline of the plurality of splines comprises a conductive layer, a polyimide layer, an adhesive layer, and a stiffening layer.

In Example 7, the electroporation catheter of any of Examples 1-6, wherein at least one ablation electrode of the one or more ablation electrodes comprises a flexible circuit, and at least one mapping electrode of the one or more mapping electrodes comprises a flexible circuit.

In Example 8, the electroporation catheter of any of Examples 1-7, wherein the one or more ablation electrodes include an internal electrode disposed on an internal component, wherein the internal component is disposed in a cavity formed by the plurality of splines.

In Example 9, the electroporation catheter of Example 3, wherein the one or more ablation electrodes comprises a tip electrode disposed on a distal end of the electrode assembly and a plurality of spline electrodes disposed on the plurality of splines.

In Example 10, the electroporation catheter of Example 9, wherein the tip electrode has a tip electrode surface area, wherein the plurality of spline electrodes have a spline electrode surface area being a summation of surface areas of the plurality of spline electrodes, wherein the tip electrode surface area is in the range of 50% to 120% of the spline electrode surface area.

In Example 11, the electroporation catheter of any of Examples 1-10, wherein the electrode assembly has a proximal end opposing the distal end, the electroporation catheter further comprising a ring electrode disposed at the proximal end of the electrode assembly.

In Example 12, an electroporation ablation system for treating target tissue in a patient, the electroporation ablation system comprising: a catheter including an electrode assembly, the electrode assembly comprising: one or more ablation electrodes configured to generate electric fields proximate to the target tissue in response to a plurality of electrical pulse sequences delivered in a plurality of therapy sections; and; one or more mapping electrodes configured measure electrical signals; a controller coupled to the catheter and configured to receive the measured electrical signals from the one or more mapping electrodes; and an electroporation generator operatively coupled to the catheter and the controller.

In Example 13, the electroporation ablation system of Example 12, wherein the electrode assembly further comprises a plurality of splines, and wherein at least a part of the one or more mapping electrodes are disposed on the plurality of splines.

In Example 14, the electroporation ablation system of Example 13, wherein the one or more ablation electrodes comprises: a tip electrode disposed on a distal end of the electrode assembly; and a plurality of spline electrodes disposed on the plurality of splines.

In Example 15, the electroporation ablation system of Example 14, wherein the tip electrode has a tip electrode surface area, wherein the plurality of spline electrodes have a spline electrode surface area being a summation of surface areas of the plurality of spline electrodes, wherein the tip electrode surface area is in the range of 50% to 120% of the spline electrode surface area.

In Example 16, an electroporation catheter comprises an electrode assembly. The electrode assembly comprises one or more ablation electrodes configured to generate electric fields proximate to target tissue in response to a plurality of electrical pulse sequences delivered in a plurality of therapy sections, and one or more mapping electrodes configured to measure electrical signals of a heart, the measured electrical signals being used to create an electro-anatomical map.

In Example 17, the electroporation catheter of Example 16 further comprises a catheter shaft having a proximal end and a distal end, wherein the electrode assembly extends from the distal end of the catheter shaft.

In Example 18, the electroporation catheter of Example 17, wherein the electrode assembly further comprises a plurality of splines.

In Example 19, the electroporation catheter of Example 18, wherein a mapping electrode of the one or more mapping electrodes is disposed on a first surface of a spline of the plurality of splines.

In Example 20, the electroporation catheter of Example 19, wherein an ablation electrode of the one or more ablation electrodes is disposed on a second surface of the spline of the plurality of splines.

In Example 21, the electroporation catheter of Example 20, wherein the ablation electrode is disposed further from the distal end of the catheter shaft than the mapping electrode.

In Example 22, the electroporation catheter of Example 18, wherein each spline of the plurality of splines comprises a conductive layer, a polyimide layer, an adhesive layer, and a stiffening layer.

In Example 23, the electroporation catheter of Example 16, wherein at least one ablation electrode of the one or more ablation electrodes comprises a flexible circuit.

In Example 24, the electroporation catheter of Example 16, wherein at least one mapping electrode of the one or more mapping electrodes comprises a flexible circuit.

In Example 25, the electroporation catheter of Example 16, wherein the one or more mapping electrodes comprise an even number of mapping electrodes.

In Example 26, the electroporation catheter of Example 18, wherein the one or more ablation electrodes include an internal electrode disposed on an internal component, wherein the internal component is disposed in a cavity formed by the plurality of splines.

In Example 27, the electroporation catheter of Example 18, wherein the one or more ablation electrodes comprises a tip electrode disposed on a distal end of the electrode assembly and a plurality of spline electrodes disposed on the plurality of splines.

In Example 28, the electroporation catheter of Example 27, wherein the tip electrode has a tip electrode surface area, wherein the plurality of spline electrodes have a spline electrode surface area being a summation of surface areas of the plurality of spline electrodes, wherein the tip electrode surface area is in the range of 90% to 110% of the spline electrode surface area.

In Example 29, the electroporation catheter of Example 16, wherein the electrode assembly has a proximal end opposing the distal end, the electroporation catheter further comprising a ring electrode is disposed at the proximal end of the electrode assembly.

In Example 30, the electroporation catheter of Example 20, wherein the first surface is an inner surface of the spline of the plurality of splines and the second surface is an outer surface of the spline of the plurality of splines.

In Example 31, a method of treating target tissue in a patient comprises disposing a mapping and ablation catheter proximate to the target tissue, the mapping and ablation catheter comprising one or more ablation electrodes and one or more mapping electrodes; collecting electrical signals by one or more mapping electrodes; creating an electro-anatomical map of an area proximate to the target tissue based on the collected electrical signals; and deploying electroporation pulses to one or more ablation electrodes, and generating electric fields proximate to the target tissue.

In Example 32, the method of Example 31, wherein the collecting electrical signals via one or more mapping electrodes comprises collecting electrical signals by the one or more mapping electrodes before deploying electroporation pulses to one or more ablation electrodes.

In Example 33, the method of Example 31, wherein the collecting electrical signals via one or more mapping electrodes comprises collecting electrical signals by the one or more mapping electrodes after deploying electroporation pulses to one or more ablation electrodes.

In Example 34, an electroporation ablation system for treating target tissue in a patient, the electroporation ablation system comprises a catheter including an electrode assembly, a controller configured to receive the measured electrical signals from the one or more mapping electrodes, and an electroporation generator operatively coupled to the catheter and the controller. The electrode assembly comprises one or more ablation electrodes configured to generate electric fields proximate to target tissue in response to a plurality of electrical pulse sequences delivered in a plurality of therapy sections, and one or more mapping electrodes configured to measure electrical signals of a heart, the measured electrical signals being used to create an electro-anatomical map.

In Example 35, the electroporation ablation system of Example 34, wherein the electrode assembly further comprises a plurality of splines.

While multiple embodiments are disclosed, still other embodiments of the present invention will become apparent to those skilled in the art from the following detailed description, which shows and describes illustrative embodiments of the invention. Accordingly, the drawings and detailed description are to be regarded as illustrative in nature and not restrictive.

### BRIEF DESCRIPTION OF THE DRAWINGS

FIG. 1 is a diagram illustrating an exemplary clinical setting for treating a patient and for treating a heart of the patient, using an electrophysiology system, in accordance with embodiments of the subject matter of the disclosure.
FIGS. 2A-2B are perspective schematic views showing the distal end of examples of mapping and ablation catheters, in accordance with embodiments of the subject matter of the disclosure.
FIG. 3 is a schematic view showing the distal end of an example mapping and ablation catheter, in accordance with embodiments of the subject matter of the disclosure.
FIG. 4 is a schematic view of an example electroporation ablation catheter, in accordance with embodiments of the subject matter of the disclosure.
FIG. 5 is an illustrative example of a layer structure for a spline, in accordance with embodiments of the subject matter of the disclosure.
FIG. 6 is an example flow diagram illustrating a method of treating target tissue in a patient, in accordance with embodiments of the subject matter of the disclosure.

While the invention is amenable to various modifications and alternative forms, specific embodiments have been shown by way of example in the drawings and are described in detail below. The intention, however, is not to limit the invention to the particular embodiments described. On the contrary, the invention is intended to cover all modifications, equivalents, and alternatives falling within the scope of the invention as defined by the appended claims.

### DETAILED DESCRIPTION

The following detailed description is exemplary in nature and is not intended to limit the scope, applicability, or configuration of the invention in any way. Rather, the following description provides some practical illustrations for implementing exemplary embodiments of the present invention. Examples of constructions, materials, and/or dimensions are provided for selected elements. Those skilled in the art will recognize that many of the noted examples have a variety of suitable alternatives.

As the terms are used herein with respect to measurements (e.g., dimensions, characteristics, attributes, components, etc.), and ranges thereof, of tangible things (e.g., products, inventory, etc.) and/or intangible things (e.g., data, electronic representations of currency, accounts, information, portions of things (e.g., percentages, fractions), calculations, data models, dynamic system models, algorithms, parameters, etc.), "about" and "approximately" may be used, interchangeably, to refer to a measurement that includes the stated measurement and that also includes any measurements that are reasonably close to the stated measurement, but that may differ by a reasonably small amount such as will be understood, and readily ascertained, by individuals having ordinary skill in the relevant arts to be attributable to measurement error; differences in measurement and/or manufacturing equipment calibration; human error in reading and/or setting measurements; adjustments made to optimize performance and/or structural parameters in view of other measurements (e.g., measurements associated with other things); particular implementation scenarios; imprecise adjustment and/or manipulation of things, settings, and/or measurements by a person, a computing device, and/or a machine; system tolerances; control loops; machine-learning; foreseeable variations (e.g., statistically insignificant variations, chaotic variations, system and/or model instabilities, etc.); preferences; and/or the like.

Although illustrative methods may be represented by one or more drawings (e.g., flow diagrams, communication flows, etc.), the drawings should not be interpreted as implying any requirement of, or particular order among or between, various steps disclosed herein. However, certain some embodiments may require certain steps and/or certain orders between certain steps, as may be explicitly described herein and/or as may be understood from the nature of the steps themselves (e.g., the performance of some steps may depend on the outcome of a previous step). Additionally, a "set," "subset," or "group" of items (e.g., inputs, algorithms, data values, etc.) may include one or more items, and, similarly, a subset or subgroup of items may include one or more items. A "plurality" means more than one.

As used herein, the term "based on" is not meant to be restrictive, but rather indicates that a determination, identification, prediction, calculation, and/or the like, is performed by using, at least, the term following "based on" as an input. For example, predicting an outcome based on a particular piece of information may additionally, or alternatively, base the same determination on another piece of information.

Irreversible electroporation (IRE) uses high voltage, short (e.g., 100 microseconds or shorter) pulses to kill cells through apoptosis. IRE can be targeted to kill myocardium, sparing other adjacent tissues including the esophageal vascular smooth muscle and endothelium. During the course of IRE therapy sections, it is desirable for a catheter that can both create an electro-anatomical map and generate electrical ablation fields in response to IRE pulse trains, for example, for cost sensitive cases. In other words, there is need for a catheter that includes multiple mapping electrodes as well as multiple ablation electrodes to receive IRE pulse trains.

At least some embodiments of the present disclosure are directed to systems and methods for creating an electro-anatomical map using the same electroporation catheter during IRE treatment. In some embodiments, an electroporation catheter includes an electrode assembly comprising one or more ablation electrodes and one or more mapping electrodes. In certain embodiments, the one or more ablation electrodes configured to generate electric fields proximate to target tissue in response to a plurality of electrical pulse sequences delivered in a plurality of therapy sections. In certain embodiments, the one or more mapping electrodes configured to measure electrical signals of a heart, the measured electrical signals being used to create an electro-anatomical map.

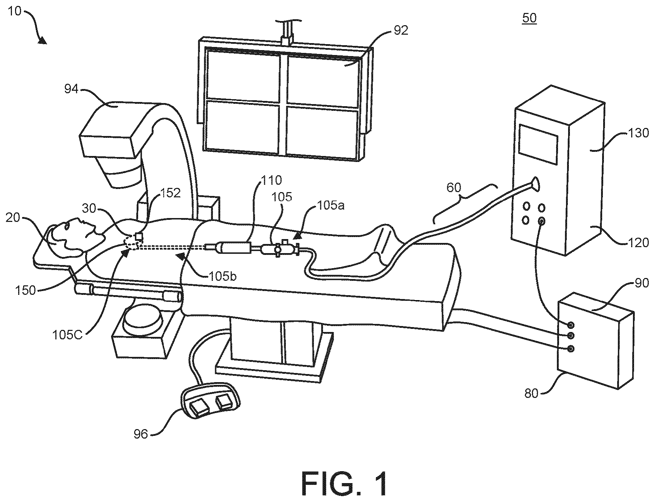

FIG. 1 is a diagram illustrating an exemplary clinical setting 10 for treating a patient 20 and for treating a heart 30 of the patient 20 using an electrophysiology system 50, in accordance with embodiments of the subject matter of the disclosure. The electrophysiology system 50 includes an electroporation device 60, a mapping controller 90, and a display 92. In some embodiments, the electroporation device 60 includes an electroporation generator 130, a controller 120, an electroporation catheter 105, and an introducer sheath 110. Also, the clinical setting 10 includes additional equipment such as imaging equipment 94 (represented by the C-arm) and various controller elements, such as a foot controller 96, configured to allow an operator to control various aspects of the electrophysiology system 50. As will be appreciated by the skilled artisan, the clinical setting 10 may have other components and arrangements of components that are not shown in FIG. 1.

In embodiments, the electroporation device 60 is configured to deliver electric field energy to target tissue in the patient's heart 30 to create tissue apoptosis, rendering the tissue incapable of conducting electrical signals. The controller 120 is configured to control functional aspects of the electroporation device 60. In embodiments, the electroporation generator 130 is operable as a pulse generator for generating and supplying pulse sequences to the electroporation catheter 105.

In embodiments, the introducer sheath 110 is operable to provide a delivery conduit through which the electroporation catheter 105 may be deployed to the specific target sites within the patient's heart 30. It will be appreciated, however, that the introducer sheath 110 is illustrated and described herein to provide context to the overall electrophysiology system 50.

In the illustrated embodiment, the electroporation catheter 105 includes a handle 105a, a shaft 105b, and an electrode assembly 150, which is described further hereinafter. The handle 105a is configured to be operated by a user to position the electrode assembly 150 at the desired anatomical location. The shaft 105b has a distal end 105c and generally defines a longitudinal axis of the electroporation catheter 105. As shown, the electrode assembly 150 is located at or proximate the distal end 105c of the shaft 105b. In embodiments, the electrode assembly 150 is electrically coupled to the electroporation generator 130, to receive electrical pulse sequences or pulse trains, thereby selectively generating electric fields for ablating the target tissue by irreversible electroporation.

In embodiments, as shown in FIG. 1, the electrode assembly 150 includes one or more mapping electrodes 152. The mapping electrodes 152 are configured to be used to generate, and display via the display 92, detailed three-dimensional geometric anatomical maps or representations of the cardiac chambers as well as electro-anatomical maps in which cardiac electrical activity of interest is superimposed on the geometric anatomical maps.

In some embodiments, the one or more mapping electrodes 152 on the electroporation catheter 105 can measure electrical signals and generate output signals that can be processed by the mapping controller 90 to generate an electro-anatomical map. In some instances, electro-anatomical maps are generated before ablation for determining the electrical activity of the cardiac tissue within a chamber of interest. In some instances, electro-anatomical maps are generated after ablation in verifying the desired change in electrical activity of the ablated tissue and the chamber as a whole. The mapping electrodes 152 may also be used to determine the position of the catheter 105 in three-dimensional space within the body. For example, when the operator moves the distal end of the catheter 105 within a cardiac chamber of interest, the boundaries of catheter movement can be used by the controller 90, which may include or couple to a mapping and navigation system, to form the anatomy of the chamber. The chamber anatomy may be used to facilitate navigation of the catheter 105 without the use of ionizing radiation such as with fluoroscopy, and for tagging locations of ablations as they are completed in order to guide spacing of ablations and aid the operator in fully ablating the anatomy of interest.

According to embodiments, various components (e.g., the mapping controller 90, the controller 120) of the electrophysiological system 50 may be implemented on one or more computing devices. A computing device may include any type of computing device suitable for implementing embodiments of the disclosure. Examples of computing devices include specialized computing devices or general-purpose computing devices such as workstations, servers, laptops, portable devices, desktop, tablet computers, hand-held devices, general-purpose graphics processing units (GPGPUs), and the like, all of which are contemplated within the scope of FIG. 1 with reference to various components of the system 50.

In some embodiments, a computing device includes a bus that, directly and/or indirectly, couples the following devices: a processor, a memory, an input/output (I/O) port, an I/O component, and a power supply. Any number of additional components, different components, and/or combinations of components may also be included in the computing device. The bus represents what may be one or more busses (such as, for example, an address bus, data bus, or combination thereof). Similarly, in some embodiments, the computing device may include a number of processors, a number of memory components, a number of I/O ports, a number of I/O components, and/or a number of power supplies. Additionally, any number of these components, or combinations thereof, may be distributed and/or duplicated across a number of computing devices.

In some embodiments, the system 50 includes memory (not illustrated). The memory includes computer-readable media in the form of volatile and/or nonvolatile memory, transitory and/or non-transitory storage media and may be removable, nonremovable, or a combination thereof. Media examples include Random Access Memory (RAM); Read Only Memory (ROM); Electronically Erasable Programmable Read Only Memory (EEPROM); flash memory; optical or holographic media; magnetic cassettes, magnetic tape, magnetic disk storage or other magnetic storage devices; data transmissions; and/or any other medium that can be used to store information and can be accessed by a computing device such as, for example, quantum state memory, and/or the like. In some embodiments, the memory stores computer-executable instructions for causing a processor (e.g., the controller 120) to implement aspects of embodiments of system components discussed herein and/or to perform aspects of embodiments of methods and procedures discussed herein.

Computer-executable instructions may include, for example, computer code, machine-useable instructions, and the like such as, for example, program components capable of being executed by one or more processors associated with a computing device. Program components may be programmed using any number of different programming environments, including various languages, development kits, frameworks, and/or the like. Some or all of the functionality contemplated herein may also, or alternatively, be implemented in hardware and/or firmware.

In some embodiments, the memory may include a data repository may be implemented using any one of the configurations described below. A data repository may include random access memories, flat files, XML files, and/or one or more database management systems (DBMS) executing on one or more database servers or a data center. A database management system may be a relational (RDBMS), hierarchical (HDBMS), multidimensional (MDBMS), object oriented (ODBMS or OODBMS) or object relational (ORDBMS) database management system, and the like. The data repository may be, for example, a single relational database. In some cases, the data repository may include a plurality of databases that can exchange and aggregate data by data integration process or software application. In an exemplary embodiment, at least part of the data repository may be hosted in a cloud data center. In some cases, a data repository may be hosted on a single computer, a server, a storage device, a cloud server, or the like. In some other cases, a data repository may be hosted on a series of networked computers, servers, or devices. In some cases, a data repository may be hosted on tiers of data storage devices including local, regional, and central.

Various components of the system 50 can communicate via or be coupled to via a communication interface, for example, a wired or wireless interface. The communication interface includes, but not limited to, any wired or wireless short-range and long-range communication interfaces. The wired interface can use cables, umbilicals, and the like. The short-range communication interfaces may be, for example, local area network (LAN), interfaces conforming known communications standard, such as Bluetooth^{®} standard, IEEE 802 standards (e.g., IEEE 802.11), a ZigBee^{®} or similar specification, such as those based on the IEEE 802.15.4 standard, or other public or proprietary wireless protocol. The long-range communication interfaces may be, for example, wide area network (WAN), cellular network interfaces, satellite communication interfaces, etc. The communication interface may be either within a private computer network, such as intranet, or on a public computer network, such as the internet.

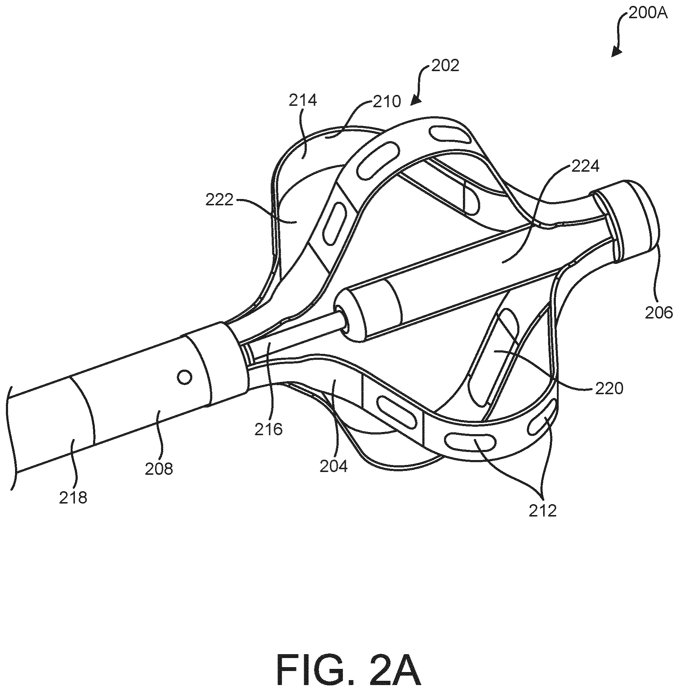

FIGS. 2A-2B are perspective schematic views showing the distal end of examples of mapping and ablation catheters, in accordance with embodiments of the subject matter of the disclosure. As shown in FIG. 2A, the mapping and ablation catheter 200A includes an electrode assembly 202 deployed in a radially extended state. The electrode assembly 202 may include one or more splines 204 and an end cap 206. Each of the splines 204 may extend between the distal end 208 of the mapping and ablation catheter 200A and the end cap 206, and forms an interior cavity 222. Each of the splines 204 may include a flexible polymer substrate 210, a plurality of mapping electrodes 212 and a plurality of ablation electrodes 220. In some embodiments, the flexible polymer substrate 210 may include a stiffening element 214. The stiffening element 214 may be, for example, a layer of nitinol. The stiffening element 214 may produce a force in each of the splines 204 that biases the electrode assembly 202 toward an undeployed state or configuration (not shown). In some embodiments, there may be 2-8 splines 204 connected to the distal end 208 of the mapping and ablation catheter 200A. In some embodiments, there may be more than 8 splines connected to the distal end 208 of the mapping and ablation catheter 200A. The skilled artisan will recognize that the spline catheter construction is well known, and the particular details of the number of splines are not of critical importance to the present disclosure.

In some embodiments, the mapping electrodes 212 may be mapping electrodes, and are configured to measure electrical signals of a heart, the measured electrical signals being used to create an electro-anatomical map. In some instances, at least a part of the one or more mapping electrodes 212 are disposed on the outer surface of the one or more splines 204. In some embodiments, the one or more mapping electrodes 212 include flexible circuits. In some embodiments, the electrode assembly 202 includes an even number of mapping electrodes 212. In some embodiments, the electrode assembly 202 may include any number of mapping electrodes 212 ranging from any numbers between 6 to 64. In some embodiments, there may be 2 mapping electrodes 212 on each spline. In some embodiments, there may be more than 2 mapping electrodes 212 on each spline (e.g., 4, 6, 8, etc.) In some embodiments, for example, for a catheter having a diameter of 2.667 millimeters (e.g., an 8 French catheter) with 64 mapping electrodes, the mapping electrodes 212 may be approximately 0.45 mm in width and 0.95 mm in length, and has a radius of 0.23 mm. In some embodiments, the dimensions of the mapping electrodes 212 may be smaller to provide higher mapping resolution. In some embodiments, the dimensions of the mapping electrodes 212 may be larger in diluting electric fields created when applying voltages. Depending on manufacturing capabilities, cost, current densities, ability of the electrode material to withstand the current densities, spline width, various sizes of mapping electrodes 212 may be used in the catheter 200A. In some examples, the spline width can be associated with the number of splines for a given catheter diameter.

As shown in FIG. 2A, the deployment shaft 216 may extend from the end cap 206 and into the catheter shaft 218 at the distal end 208. In some embodiments, the end cap 206 may be an electrode for cardiac stimulation or tissue ablation or mapping. Furthermore, the tip electrode may be a series of surfaces that an act as a single electrode for application of therapy or a multitude of electrodes for mapping. In some embodiments, the end cap 206 may contain a navigation sensor for use in determining the location of the electrode assembly 202 within the body. In some designs, the ablation electrodes 220 have rounded corners. In some examples, the ablation electrode 220 has a selected arc at each corner. In some implementations, the end cap 206 includes an ablation electrode 220.

In some embodiments, the electrode assembly 202 includes an internal component 224 disposed at the interior cavity 222 of the electrode assembly 202. The deployment shaft 216 may be connected to the internal component 224. In some instances, the internal component 224 may include a navigation sensor. In some embodiments, the electrode assembly further includes one or more ablation electrodes 220. In some examples, a part or all of the ablation electrodes 220 are disposed on the inner surface of the one or more splines 204. In some examples, at least one of the ablation electrodes 220 is disposed on the outer surface of the one or more splines 204. The one or more ablation electrodes 220 are configured to generate electric fields in target tissue in response to a plurality of electrical pulse sequences delivered in a plurality of therapy sections. In some embodiments, the one or more ablation electrodes 220 include flexible circuits. In some designs, each spline 204 includes one ablation electrode 220 disposed on the spline. In some designs, each spline 204 includes one or more mapping electrodes 212 disposed on the splines.

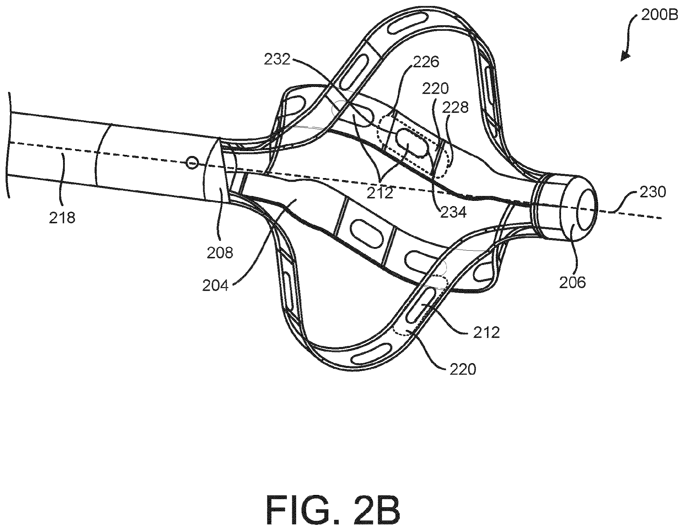

As shown in FIG. 2B, one or more ablation electrodes 220 are disposed on an interior surface of a spline 204 and one or more mapping electrodes 212 are disposed on an outer surface of the spline 204. In some embodiments, the one or more ablation electrodes 220 are disposed further from the distal end 208 of the catheter shaft 218 than the one or more mapping electrodes 212. In some embodiments, at least one ablation electrode 220 is disposed closer to an end cap 206 than all mapping electrodes 212 disposed on splines 204. In some implementations, each mapping electrode 212 on a spline 204 is disposed closer to the distal end 208 than a respective ablation electrode 220.

In some cases, an ablation electrode 220 includes a first edge 226 and a second edge 228 opposing to the first edge 226 along a longitudinal axis 230 of the mapping and ablation catheter 200B, where the second edge 228 is closer to the end cap 206. In some cases, a mapping electrode 212 includes a first edge 232 and a second edge 234 opposing to the first edge 232 along the longitudinal axis 230. In some embodiments, the second edge 228 of the ablation electrode 220 is further away from the distal end 208 of the catheter shaft 218 than the second edge 234 of the mapping electrode 212. As shown, for example, the second edge 228 of the ablation electrode 220 is further from the distal end 208 of the catheter shaft 218 than the second edge 234 of the mapping electrode 212. In some embodiments, the second edge 228 of the ablation electrode 220 is closer to the distal end 208 of the catheter shaft 218 than the first edge 232 of the mapping electrode 212. In other embodiments (not shown), the first edge 226 of the ablation electrode 220 is further away from the distal end 208 of the catheter shaft 218 than the first edge 232 of the mapping electrode 212.

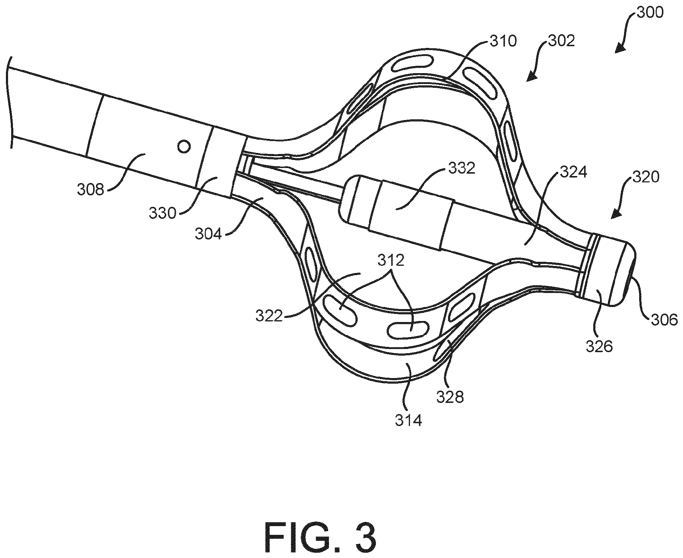

FIG. 3 is a schematic view of the distal end of an example mapping and electroporation catheter 300, in accordance with embodiments of the subject matter of the disclosure. As shown in FIG. 3, the catheter 300 includes an electrode assembly 302 are deployed in a radially extended state. The electrode assembly 302 may include one or more splines 304 and an end cap 306. Each of the splines 304 may extend between the distal end 308 of the catheter 300 and the end cap 306, and forms an interior cavity 322. Each of the splines 304 may include a flexible polymer substrate 310, a plurality of mapping electrodes 312, and one or more ablation electrodes 328. In some embodiments, the flexible polymer substrate 310 may include a stiffening element 314. The stiffening element 314 may be, for example, a layer of nitinol. The stiffening element 314 may produce a force in each of the splines 304 that biases the electrode assembly 302 toward an undeployed state or configuration (not shown). In some embodiments, there may be 2-8 splines 304 connected to the distal end 308 of the catheter 300. In some embodiments, there may be more than 8 splines connected to the distal end 308 of the catheter 300. The skilled artisan will recognize that the spline catheter construction is well known, and the particular details of the number of splines are not of critical importance to the present disclosure.

In some embodiments, as shown in FIG. 3, the one or more ablation electrodes 320 include a tip electrode 326 disposed on the distal end (e.g., the end cap 306) of the electrode assembly 302. In some embodiments, the one or more ablation electrodes 320 include a tip electrode 326 disposed on the end cap 306 of the electrode assembly 302 and a plurality of spline electrodes 328 disposed on the plurality of splines 304. In one example, the electrode assembly 302 includes four (4) spline electrodes 328 and a tip electrode 326.

In some embodiments, the ablation electrodes 328 located on the splines 314 may be supplemented or replaced by one or more additional ring electrodes 332 located on the internal component 324. If the electrode 332 replaces the ablation electrodes 328, the surface area of the electrode 332 may be approximately equal to the surface area of the tip electrode 326. If the electrode 332 supplements the ablation electrodes 328, the surface area of the electrode 332 may be a fraction of the surface area of the tip electrode 326. In some embodiments, the electrode 332 may include multiple ring electrodes (not shown) to both replace and supplement the electrodes 328; the number of rings may be energized as needed during the pulse train.

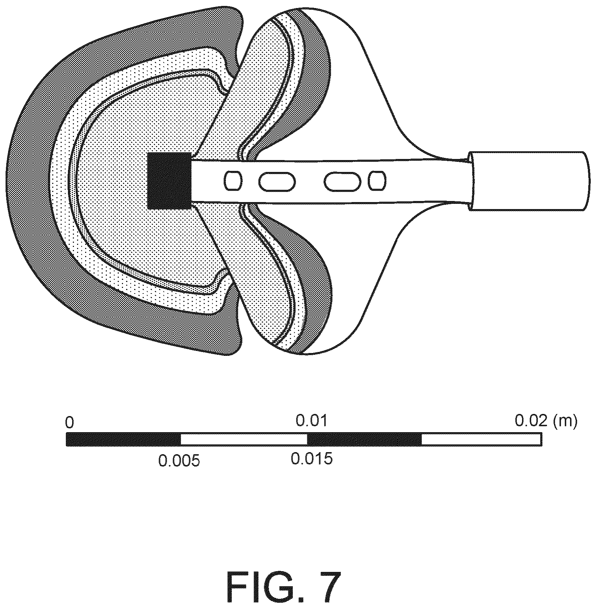

The tip electrode 326 has a ring surface area, wherein the plurality of spline electrodes 328 have a spline electrode surface area being a summation of surface areas of the plurality of spline electrodes 328. In some examples, the surface area of the tip electrode 326 is in the range of 90% to 110% of the spline electrode surface area. For example, if the surface area of the tip electrode 326 is 21.5 mm², the summation of the surface area of the spline electrodes 328 is in the range of 19.35 to 23.65 mm². In some examples, the surface area of the tip electrode 326 is in the range of 80% to 120% of the spline electrode surface area. In some designs, the surface area of the tip electrode 326 is in the range of 50% to 150% of the spline electrode surface area. In certain examples, the surface area of the tip electrode 326 is in the range of 50% to 120% of the spline electrode surface area. In some implementations, the surface area of the tip electrode being similar to spline electrode surface area can reduce arching and improve the effectiveness of the ablation functionality of the mapping and ablation catheter 300. FIG. 7 illustrates one example of electroporation field generated by one catheter having a tip electrode at the end cap.

In some embodiments, the electrode assembly 302 has a proximal ring electrode 330 disposed on the proximal end of the electrode assembly 302. In one example, the ablation electrodes of the electrode assembly 302 include four (4) spline electrodes 328, a tip electrode 326, a ring electrode 332 and a proximal ring electrode 330. In certain embodiments, the ablation electrodes include anode electrode(s) and cathode(s), where the overall surface area of anode electrode(s) (i.e., a summation of surface areas of respective electrodes) is similar to the overall surface area of cathode electrode(s). For example, a difference between the overall surface area of anode electrode(s) and the overall surface area of cathode electrode(s) is within 50% of the overall surface area of anode electrode(s). As another example, a difference between the overall surface area of anode electrode(s) and the overall surface area of cathode electrode(s) is within 20% of the overall surface area of anode electrode(s). As yet another example, a difference between the overall surface area of anode electrode(s) and the overall surface area of cathode electrode(s) is within 10% of the overall surface area of anode electrode(s).

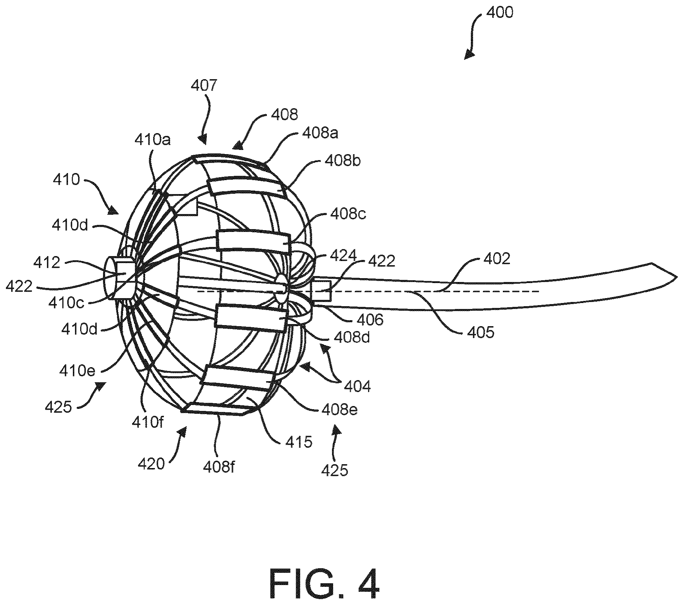

FIG. 4 is a schematic view of an example electroporation ablation catheter 400, in accordance with embodiments of the subject matter of the disclosure. As shown in FIG. 4, the catheter 400 includes a catheter shaft 402 with a longitudinal axis 405 and having a distal end 406. As used herein, a longitudinal axis refers to a line passing through the centroid of the cross sections of an object. The catheter 400 further includes an electrode assembly 407. In some embodiments, the electrode assembly 407 extends from the distal end 406 of the catheter shaft 402. In embodiments, the electrode assembly 407 is configured to assume a first collapsed state and a second expanded state. In some cases, the electrode assembly 407 includes an expandable component 420, a plurality of mapping electrodes 422 and a plurality of ablation electrodes 425 disposed on the expandable component 420. The expandable component 420 can be collapsed in the first state and expanded in the second state.

In some embodiments, the electrode assembly 407 includes a plurality of splines 404 forming an interior cavity 415 and an inflatable balloon (not shown) disposed in the cavity 415. In such embodiment, the plurality of splines 404 and the balloon collectively form the expandable component 420.

In some embodiments, the catheter 400 is configured to receive an ablative energy (e.g., electroporation pulse) at the plurality of ablation electrodes 425 and generate an electric field at the ablation electrodes 425. In one embodiment, the electric field has an electric field strength sufficient to ablate a target tissue via irreversible electroporation. In some embodiments, the plurality of mapping electrodes 422 and the plurality of ablation electrodes 425 includes a first group of electrodes 408 and a second group of electrodes 410. In some cases, the first group of electrodes 408 disposed at the circumference of the plurality of splines 404 and the second group of electrodes 410 disposed adjacent the distal end 412 of the catheter 400. In some cases, the first group of electrodes 408 are referred to as proximal electrodes, and the second group of electrodes 410 are referred to as distal electrodes, where the distal electrodes 410 are disposed closer to the distal end 412 of the electroporation ablation catheter 400 than the proximal electrodes 408. In some implementations, the ablation electrodes 425 can include a thin film of an electro-conductive or optical ink. The ink can be polymer-based. The ink may additionally comprise materials such as carbon and/or graphite in combination with conductive materials. The electrode can include a biocompatible, low resistance metal such as silver, silver flake, gold, and platinum which are additionally radiopaque.

In some implementations, at least a part of or all electrodes in the first group of electrodes 408 and/or at least a part of or all the electrodes in the second group of electrodes 410 are configured to conduct electricity and to be operably connected to a controller and an ablative energy generator (see FIG. 1). In certain implementations, at least a part of or all electrodes in the first group of electrodes 408 and/or at least a part of or all the electrodes in the second group of electrodes 410 are configured to measure electrical signals (e.g., mapping electrodes) and to be operably connected to a controller. In embodiments, one or more of the electrodes in the first group of electrodes 408 and the second group of electrodes 410 includes flex circuits.

Electrodes in the first group of electrodes 408 are spaced apart from electrodes in the second group of electrodes 410. The first group of electrodes 208 includes electrodes 408a-408f and the second group of electrodes 410 includes electrodes 410a-410f. Also, electrodes in the first group of electrodes 408, such as electrodes 408a-408f, are spaced apart from one another and electrodes in the second of electrodes 410, such as electrodes 410a-410f, are spaced apart from one another.

The spatial relationships and orientation of the electrodes in the first group of electrodes 408 and the spatial relationships and orientation of the electrodes in the second group of electrodes 410 in relation to other electrodes on the same catheter 400 is known or can be determined. In embodiments, the spatial relationships and orientation of the electrodes in the first group of electrodes 408 and the spatial relationships and orientation of the electrodes in the second group of electrodes 410 in relation to other electrodes on the same catheter 400 is constant, once the catheter is deployed.

In some embodiments, one or more mapping electrodes 422 may be disposed at the proximal and distal end of the electrode assembly 407. The one or more mapping electrodes 422 are configured to measure electrical signals of a heart, the measured electrical signals being used to create an electro-anatomical map.

In some embodiments (not shown), the one or more mapping electrodes may be disposed proximal or distal to the ablation electrodes on the same side of the same splines. In some embodiments (not shown), the one or more mapping electrodes may be part of a flex circuit, and disposed proximal or distal to the electrodes 425 on different sides of the same splines. In some embodiments (not shown), the one or more mapping electrodes may be disposed proximal or distal to the ablation electrodes on different splines. When the mapping electrodes and ablation electrodes are disposed on different or alternating splines, high voltage and low voltage electrical pathways can be kept separate and better insulated from each other. In some embodiments, the surface area of mapping electrodes is smaller than the surface area of ablation electrodes to minimize far field sensed electrical signals.

In some embodiments, the group of electrodes 408 may be ablation electrodes whereas the group of electrodes 410 may be mapping electrodes. In some embodiments, the group of electrodes 408 may be mapping electrodes whereas the group of electrodes 410 may be ablation electrodes. In some embodiments, where electrodes 408 and 410 are both ablation electrodes, the mapping electrodes may be disposed on an internal component 424 as ring electrodes. In some embodiments, the ablation electrodes may include a tip electrode (e.g., at the distal end 412 of the catheter 400), one or more ring electrodes (e.g., at the distal end 406 of the catheter shaft 402), and/or one or more spline electrodes, and the mapping electrodes may be disposed on the splines. In some embodiments, the mapping electrodes and the ablation electrodes are disposed on different splines. For example, electrodes 408a, 408c, 408e, 410a, 410c, and 410e are ablation electrodes; and electrodes 408b, 408d, 408f, 410b, 410d, and 410f are mapping electrodes.

In some embodiments, one or more mapping electrodes 422 may be disposed on an outer surface of the expandable component 420 (not shown). In some embodiments, at least a part or all of the groups of electrodes 408, 410 may be disposed on an inner surface of the expandable component 420.

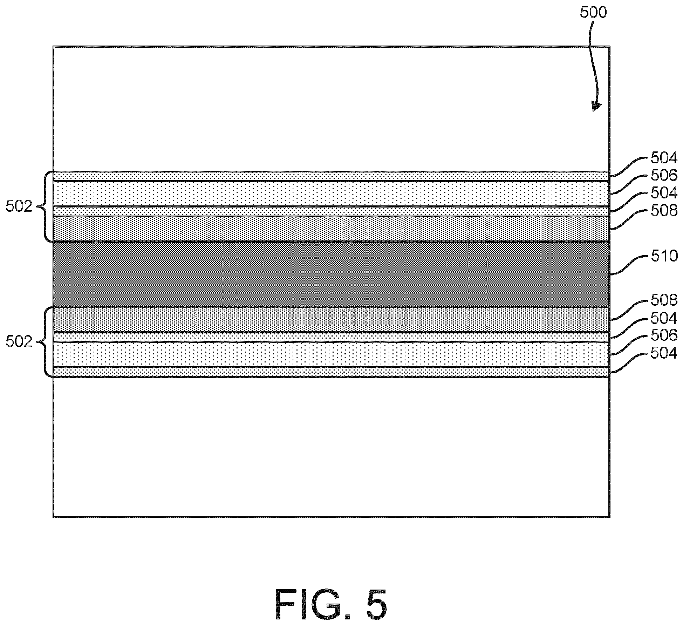

FIG. 5 depicts one example of a layer structure 500 forming the one or more splines (e.g., the splines 204 in FIGS. 2A and 2B, the splines 304 in FIG. 3), in accordance with certain embodiments of the present disclosure. In some embodiments, the one or more layers 500 include one or more flexible circuit layers 502. Each flexible circuit includes one or more conductive layers 504 and a polyimide flexible circuit material layer 506. In some embodiments, the flexible circuit layers 502 includes an adhesive layer 508. In some embodiments, the flexible circuit does not include an adhesive layer 508. In some embodiments, the one or more layers 500 include a stiffening layer 510.

In some examples, the stiffening layer 510 includes material that is flexible and non-conductive (e.g., plastics). In some examples, the stiffening layer 510 includes polyimide. In some examples, the stiffening layer 510 includes material that is stiff and conductive (e.g., nitinol) so that the splines have a longer aspect ratio.

In some examples, the conductive layer includes copper. In some examples, the conductive layer includes gold embedded within liquid crystal polymer. In some examples, the conductive layer includes a noble metal (e.g., platinum). In some examples, the conductive layer includes titanium. In some examples, the conductive layer includes a copper based with a gold layer on top to protect it from corrosion. In some examples, the conductive layer may include a layer of platinum or iridium, which are subsequently oxidized to form platinum/iridium oxide to improve mapping performance. In some embodiments, a nickel layer may be added in between a copper and gold layers to retard the diffusion of the copper into the gold, thus reduce the corrosion protection offered by the gold.

In some examples, the flex circuit layers 502 may include polyimide or liquid crystal polymer (LCP) to tolerate soldering temperatures. In some examples, the flex circuit layers 502 may include glass. In some examples, the flex circuit layers 502 may include other polymers employed along with low temperature interconnect methods (e.g., solder jetting).

In some examples, the adhesive layer 508 may include acrylic, polyimide, epoxy or fluoropolymer adhesives. In some designs, a spline (e.g., the splines 204 in FIGS. 2A and 2B, the splines 304 in FIG. 3) is formed by the layer structure 500 sealed using non-conductive materials at both sides.

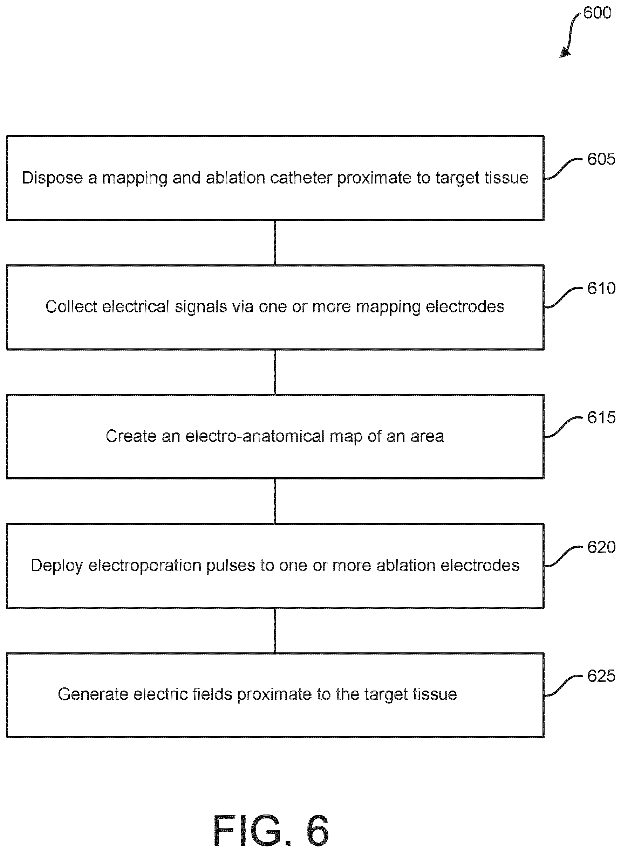

FIG. 6 is a flow chart diagram illustrating a method 600 of treating target tissue in a patient, in accordance with embodiments of the subject matter of the disclosure. Aspects of embodiments of the method 600 may be performed, for example, by an electroporation ablation system/device (e.g., the system/device 50 depicted in FIG. 1). One or more steps of method 600 are optional and/or can be modified by one or more steps of other embodiments described herein. Additionally, one or more steps of other embodiments described herein may be added to the method 600. As shown in FIG. 6, a mapping and ablation catheter may be disposed proximate to target tissue within a patient (605). In some embodiments, electrical signals are collected via one or more mapping electrodes (610). In some embodiments, an electro-anatomical map of an area is created based upon the collected electrical signals (615), for example, an electro-anatomical map of an area proximate to the target tissue. In some embodiments, electroporation pulses are deployed to one or more ablation electrodes (620). In some embodiments, electric fields are generated proximate to the target tissue (625). In certain embodiments, electrical signals are collected via one or more mapping electrodes after the ablation and the electro-anatomical map is generated or updated.

FIG. 7 shows an exemplary bipolar electric field of a mapping and ablation catheter in a deployed state, in accordance with embodiments of the subject matter of the disclosure. As shown, the electric field gives a nearly uniform depth in all tissue-facing directions distal to the tip electrode, where electroporation ablation is desired to occur, while also containing the field at proximal return electrodes within the inner surface of the splines so that more proximal tissue is not ablated where not desired.

In some embodiments, the mapping and ablation catheter may provide monopolar ablation with a surface patch. In some examples, the surface patch may be disposed at a patient's skin. In such examples, the ablation from the catheter to thea surface patch (not shown) may cause a deeper lesion and also protect circuits within the catheter from high voltages.

Various modifications and additions can be made to the exemplary embodiments discussed without departing from the scope of the present invention. For example, while the embodiments described above refer to particular features, the scope of this invention also includes embodiments having different combinations of features and embodiments that do not include all of the described features. Accordingly, the scope of the present invention is intended to embrace all such alternatives, modifications, and variations as fall within the scope of the claims, together with all equivalents thereof.

The invention relates, inter alia, to the following aspects:
1. An electroporation catheter, comprising:
   an electrode assembly comprising:
   one or more ablation electrodes configured to generate electric fields proximate to target tissue in response to a plurality of electrical pulse sequences delivered in a plurality of therapy sections; and
   one or more mapping electrodes configured to measure electrical signals of a heart, the measured electrical signals being used to create an electro-anatomical map.
2. The electroporation catheter of aspect 1, further comprising:
   a catheter shaft having a proximal end and a distal end;
   wherein the electrode assembly extends from the distal end of the catheter shaft.
3. The electroporation catheter of any of aspects 1-2, wherein the electrode assembly further comprises a plurality of splines.
4. The electroporation catheter of aspect 3, wherein a mapping electrode of the one or more mapping electrodes is disposed on a first surface of a spline of the plurality of splines, and an ablation electrode of the one or more ablation electrodes is disposed on a second surface of the spline of the plurality of splines.
5. The electroporation catheter of any of aspects 2-4, wherein the ablation electrode is disposed further from the distal end of the catheter shaft than the mapping electrode.
6. The electroporation catheter of any of aspects 3-5, wherein each spline of the plurality of splines comprises a conductive layer, a polyimide layer, an adhesive layer, and a stiffening layer.
7. The electroporation catheter of any of aspects 1-6, wherein at least one ablation electrode of the one or more ablation electrodes comprises a flexible circuit, and at least one mapping electrode of the one or more mapping electrodes comprises a flexible circuit.
8. The electroporation catheter of any of aspects 1-7, wherein the one or more ablation electrodes include an internal electrode disposed on an internal component, wherein the internal component is disposed in a cavity formed by the plurality of splines.
9. The electroporation catheter of aspect 3, wherein the one or more ablation electrodes comprises:
   a tip electrode disposed on a distal end of the electrode assembly; and
   a plurality of spline electrodes disposed on the plurality of splines.
10. The electroporation catheter of aspect 9, wherein the tip electrode has a tip electrode surface area, wherein the plurality of spline electrodes have a spline electrode surface area being a summation of surface areas of the plurality of spline electrodes, wherein the tip electrode surface area is in the range of 50% to 120% of the spline electrode surface area.
11. The electroporation catheter of any one of aspects 1-10, wherein the electrode assembly has a proximal end opposing the distal end, the electroporation catheter further comprising:
   a ring electrode disposed at the proximal end of the electrode assembly.
12. An electroporation ablation system for treating target tissue in a patient, the electroporation ablation system comprising:
   a catheter including an electrode assembly, the electrode assembly comprising:
      one or more ablation electrodes configured to generate electric fields proximate to the target tissue in response to a plurality of electrical pulse sequences delivered in a plurality of therapy sections; and;
      one or more mapping electrodes configured measure electrical signals;
   a controller coupled to the catheter and configured to receive the measured electrical signals from the one or more mapping electrodes; and
   an electroporation generator operatively coupled to the catheter and the controller.
13. The electroporation ablation system of aspect 12, wherein the electrode assembly further comprises a plurality of splines, and wherein at least a part of the one or more mapping electrodes are disposed on the plurality of splines.
14. The electroporation ablation system of aspect 13, wherein the one or more ablation electrodes comprises:
   a tip electrode disposed on a distal end of the electrode assembly; and
   a plurality of spline electrodes disposed on the plurality of splines.
15. The electroporation ablation system of aspect 14, wherein the tip electrode has a tip electrode surface area, wherein the plurality of spline electrodes have a spline electrode surface area being a summation of surface areas of the plurality of spline electrodes, wherein the tip electrode surface area is in the range of 50% to 120% of the spline electrode surface area.

## Claims

1. An electroporation catheter comprising:
a catheter shaft having a proximal end and a distal end; and
an electrode assembly extending from the distal end of the catheter shaft, the electrode assembly comprising:
a plurality of splines defining an interior cavity;
one or more ablation electrodes configured to generate electric fields proximate to the target tissue in response to a plurality of electrical pulse sequences delivered in a plurality of therapy sections, the plurality of ablation electrodes comprising:
a tip electrode disposed on a distal end of the electrode assembly; and
a plurality of spline electrodes disposed on the plurality of splines;
one or more mapping electrodes configured measure electrical signals, wherein the one or more mapping electrodes are disposed on the plurality of splines;
an internal component disposed within the interior cavity; and
one or more ring electrodes disposed on the internal component.

2. The electroporation catheter of claim 1, wherein a mapping electrode of the one or more mapping electrodes is disposed on a first surface of a spline of the plurality of splines, and an ablation electrode of the one or more ablation electrodes is disposed on a second surface of the spline of the plurality of splines

3. The electroporation catheter of claim 2, wherein the first surface is an inner surface of the spline of the plurality of splines and the second surface is an outer surface of the spline of the plurality of splines.

4. The electroporation catheter of any of claims 1-3, wherein the one or more ablation electrodes includes the one or more ring electrodes.

5. The electroporation catheter of either of any of claims 2-4, wherein the tip electrode has a tip electrode surface area, wherein the plurality of spline electrodes have a spline electrode surface area being a summation of surface areas of the plurality of spline electrodes, wherein the tip electrode surface area is in the range of 90% to 110% of the spline electrode surface area.

6. The electroporation catheter of any of claims 1-5, wherein the one or more ablation electrodes are disposed further from the distal end of the catheter shaft than the one or more mapping electrodes.

7. The electroporation catheter of any of claims 1-6, wherein at least one ablation electrode of the one or more ablation electrodes comprises a flexible circuit.

8. The electroporation catheter of any of claims 1-7, wherein each spline of the plurality of splines comprises a conductive layer, a polyimide layer, an adhesive layer, and a stiffening layer.

9. The electroporation catheter of any of claims 1-8, wherein at least one mapping electrode of the one or more mapping electrodes comprises a flexible circuit.

10. The electroporation catheter of any of claims 1-9, wherein the one or more mapping electrodes comprise an even number of mapping electrodes.

11. The electroporation catheter of any of claims 1-10, further comprising a navigation sensor.

12. The electroporation catheter of claim 11, wherein the internal component includes the navigation sensor.

13. An electroporation ablation system for treating target tissue in a patient, the electroporation ablation system comprising:
the electroporation catheter of any of claims 1-12;
a controller coupled to the electroporation catheter and configured to receive the measured electrical signals from the one or more mapping electrodes; and
an electroporation generator operatively coupled to the electroporation catheter and the controller.

14. The electroporation ablation system of claim 13, wherein the electroporation generator is configured to deliver electroporation pulses to the one or more ablation electrodes.
